# EUROPEAN PATENT SPECIFICATION

(11) **EP 0 723 768 B1**
(45) Date of publication and mention of the grant of the patent: **21.04.1999**
(21) Application number: 96201203.5
(22) Date of filing: 25.10.1993
(51) Int. Cl.: A61F 13/26

(54) **Tampon applicator**
Tamponapplikator
Applicateur de tampon

(30) Priority: 06.11.1992 US 973156; 06.11.1992 US 973072
(43) Date of publication of application: 31.07.1996
(62) Divisional of application: 93925036.1
(73) Proprietor: TAMBRANDS, INC., White Plains, New York 10604 (US)
(72) Inventor: Frayman, Max, Longmeadow, MA 01106 (US); Lindsay, Richard J., North Brookfield, MA 01535 (US); Sprague, E.Russel, Palm City, FL 34990 (US); Tomaszewski, Gina M., Monson, MA 01057 (US); Cavanaugh, Garrett, Three Rivers, MA 01080 (US)
(74) Representative: Kremer, Véronique Marie Joséphine

(56) References cited:
- EP-A- 0 066 213
- EP-A- 0 551 758
- GB-A- 2 114 448
- US-A- 3 433 225

## Description

### Background of the Invention

This invention relates to a tampon applicator, formed from a paper laminate.

Tampon applicators comprising a pair of telescopically arranged tubes have long been known in the art. Typically, these applicators comprise a tampon holder tube and a plunger tube, telescopically disposed such that a portion of the plunger tube is within the holder tube.

Some applicators have the tampon exposed at the end intended for vaginal insertion (the expulsion end), while others provide a rounded expulsion end, with the tampon covered by a plurality of segments, referred to in the art as "petals", which open during tampon expulsion. A tampon having the latter construction is disclosed in U.S. Patent No. 5,087,239, the disclosure of which is incorporated herein by reference. In this applicator, a weakening formation, e.g., a groove, is provided at the base of the segments. The weakening formation functions as a hinge, reducing the force required to bend the segments outwardly during expulsion.

However, the force required to bend the segments during expulsion may be undesirably high, even if a groove is provided at the base of the segments. This occurs particularly when a thick, stiff paper laminate, or a laminate including a layer of cellophane or polymeric film, as disclosed in copending application U.S. Serial No. 07/819,753, is used in a segment construction. The high force required may cause discomfort to the user, or may prevent the tampon from being properly expelled.

### SUMMARY OF THE INVENTION

The invention features an improved tampon applicator for insertion of a tampon into a body cavity, including a paper tampon holder tube having an expulsion end dimensioned for insertion into the body cavity and including a plurality of contiguous petal segments separated by slits, and a plunger, telescopically and slidably mounted in the holder distal to the expulsion end and adapted to expel the tampon from the holder when pushed manually into the holder. As the tampon is expelled, the segments bend open at a hinge. The segments of the improved applicator bend outwardly during expulsion with significantly reduced applied force, improving user comfort.

In one aspect, the tampon holder includes a plurality of small cuts extending circumferentially from the petal slits in the vicinity of the hinge. The cuts may take a variety of forms, so long as they narrow the circumferential extent, i.e., the width, of the petal segments. The cuts reduce the bending moment and rigidity at the hinge, both by shortening the width of the "beam" being bent, and by reducing the curvature of the "beam". In preferred embodiments, the tampon holder is a paper laminate; there is a cut at or near the base of each petal slit; the cuts have a length that effectively narrows the width of the petal segments by anywhere from about 15 to 50 percent.

In another aspect, the tampon holder includes a zone of indentation disposed on the inside of the tampon holder tube and axially overlapping the hinge, which is defined by a weakening formation (e.g, a groove or scoring) on the exterior. The zone may comprise a plurality of score lines, arranged in a variety of patterns, and the score lines may be continuous or discontinuous. In preferred embodiments, the tampon holder is a paper laminate; the score lines are combined with the circumferential cuts, both located adjacent the hinge so as to reduce the force required to bend the segments at the hinge; the score lines extend circumferentially, and one or more of the score lines are disposed slightly above (closer to the distal end of the holder tube), and one or more slightly below the weakening formation. Preferably, the depth of the score lines is from about 25 to 80 percent of the total thickness of the paper laminate.

Preferably, one or both of the circumferential cuts and interior score lines is combined with a further weakening formation at the vicinity of the hinge (e.g., a groove or scoring, which may be continuous or discontinuous), as shown in U.S. Patent No. 5,087,239.

For particularly thick or stiff paper laminates, it may be desirable to use both circumferential cuts and interior score lines in combination. For thinner paper laminates, the circumferential cuts or the score lines may be used alone, as the combination of cuts and score lines may in some instances cause the segments to rip off.

Other features and advantages of the invention will be apparent from the following description of preferred embodiments, and from the claims.

### Brief Description of the Drawings

Fig. 1 shows a tampon applicator according to one embodiment of the invention.
Fig. 2 shows a partial side cross-sectional view of the applicator of Fig. 1, enlarged to show detail. Fig. 2a shows a front view of the triangular notches which are removed from the tampon expulsion end to form the petal segments.
Fig. 3 is an enlarged view of the expulsion end of the embodiment shown in Fig. 1.
Figs. 3a-3e show the expulsion ends of tampon applicators according to some of many alternate embodiments of the invention.

### Description of the Preferred Embodiments

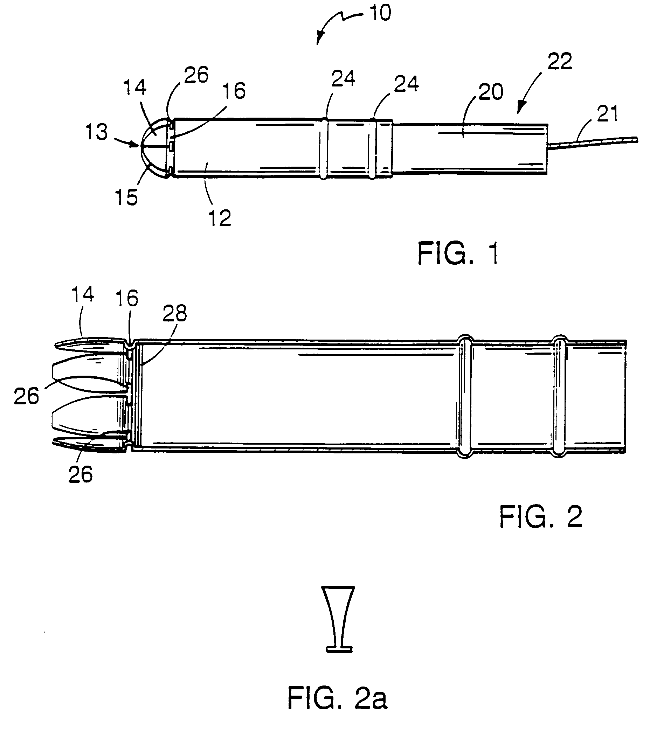

An applicator 10 according to a preferred embodiment of the invention is shown in Figs. 1 and 2. The applicator comprises tubular tampon holder 12, and plunger 20, telescopically and slidably mounted inside of tampon holder 12. A tampon 13 is retained within tampon holder 12, and its expulsion end is surrounded by contiguous petal segments 14, separated by petal slits 15. Petal segments 14 are formed by cutting out triangular notches of the paper laminate (as is known, e.g., in U.S. Patent 5,087,239). This may occur either prior to formation of the tube by stamping, in convolutely wound tampons, or after formation of a spirally wound tube. The segments are then heat-formed into a dome-shape.

In use, as plunger 20 is pressed inwardly, petal segments 14 bend open at a hinge, and the tampon is expelled through the expulsion end.

To reduce the expulsion force required to bend the petal segments open at the hinge, a weakening formation in the form of a groove 16 is embossed into the outer surface of tampon holder 12 at the hinge. Groove 16 extends circumferentially around, at, or near, the base of the segments. The weakening formation may take a variety of forms other than a groove (e.g., it may be a slot or perforation, and it may be continuous or discontinuous). Tampon withdrawal cord 21 extends out of distal end 22 of the applicator. Rings 24 are embossed into tampon holder 12 at its distal end, to provide a gripping surface for the user.

Substantial further reduction in expulsion force is achieved by providing a plurality of small circumferential cuts 26 at the vicinity of the hinge. Each cut extends circumferentially a small distance in each direction from the slit 15. The cuts reduce the bending moment and rigidity at the hinge, both by shortening the width of the "beam" being bent, and by reducing the curvature of the "beam". In a preferred embodiment, each cut is from about 0.04 (101,6.10⁻⁵m) to 0.09" (228,6.10⁻⁵m) long, about 0.01 (25,4.10⁻⁵m) to 0.03" (76.10⁻⁵m) high, and has a width approximately equal to that of slits 15.

Further reduction in expulsion force is achieved by providing a zone of indentation, preferably a plurality of score lines 28, disposed on the inside of the tampon holder, and axially overlapping the weakening formation (groove 16) on the exterior. Preferably, groove 16 is aligned in the middle of the zone of indentation (Fig. 2), but this can vary so long as the zone axially overlaps the weakening formation. Score lines 28 are provided across an area extending from slightly above to slightly below groove 16, and are spaced about 0.030" (76.10⁻⁵m) apart. In alternate embodiments, the zone of indentation could be an embossed band in which the thickness of the holder is reduced; the depth of the embossing could be uniform or varying. If the zone is made up of score lines, there may be more score lines than shown in Fig. 2 (e.g., the score lines may extend further up the segments), or fewer score lines (e.g., only one on either side of the groove), and the score lines may be spaced closer or further apart, preferably from about 0.010 (25,4.10⁻⁵m) to 0.050" (127.10⁻⁵m) apart. Preferably, the depth of indentation in the zone is from about 25 to 80 percent of the total thickness of the paper laminate. For the preferred laminate, the score lines are about 0.008 (20,32.10⁻⁵m) to 0.010" (25,4.10⁻⁵m) deep. The score lines may be arranged in many other patterns, e.g., cross-hatching in which score lines intersect, and the scoring can be either continuous or discontinuous provided there is scoring around substantially the entire inner surface of the tampon holder. The score lines may be formed by any suitable process, but are typically formed by a mandrel and rolling die process. The circumferential cuts and score lines may be used in combination (as shown in Fig. 2) or alone, depending on the stiffness of the paper and desired expulsion pressure.

A preferred tampon holder 12 is constructed from a laminate of the following layers: three bleached Kraft outer paper layers and a cellophane layer disposed on the outer tube surface. It is preferred that both the tampon holder and the plunger have this construction, although the holder and the plunger may be made from different materials, e.g., plastic or wax-coated paper, or with ground-wood paper in some of the layers, if desired.

Each of the layers is adhered to adjoining layers by adhesive, preferably a water-based adhesive, to allow the layers to delaminate when the applicator is exposed to water, thereby permitting the applicator to be disposed of by flushing. The water-based adhesive also preserves the biodegradability of the product. Suitable adhesives include, for example, Dextrin™ (vegetable-based adhesive) and polyvinyl acetate, with polyvinyl acetate being preferred for its ability to bond to cellophane under humid storage conditions. Suitable polyvinyl acetate adhesives are commercially available from Findley Adhesives, Wauwatosa, WI.

The cellophane layer may be of any grade of cellophane so long as it has adequate water resistance to maintain its integrity and remain tack-free when used in the invention, i.e. adequate to withstand humid environments for long periods of time, and to withstand insertion into the vagina, without becoming tacky or dissolving. Accordingly, the term does not refer to pure regenerated cellulose which is free from any additives or coatings, for such material has extremely low water resistance. Suitable cellophanes may contain additives or surface coatings which impart water resistance, with surface coatings being preferred. Preferred water resistant coatings are selected from the group consisting of nitrocellulose and polyvinylidene chloride, with nitrocellulose preferred for its biodegradability. A nitrocellulose coated cellophane sheet is commercially available from Flexel, Inc., Atlanta, GA. Water resistance is typically measured in terms of Water Vapor Transmission Rate (WVTR). It is preferred that the cellophane film have a WVTR of less than about 100 g/m²/day. It is preferred that the cellophane layer be thin relative to the paper layer. Preferably the thickness is less than about 0.0020 (5,08.10⁻⁵m) inches, more preferably less than 0.0009 (2,286.10⁻⁵m) inches.

It is preferred, for aesthetic reasons, that the outer paper layer be a white paper. Although any conventional paper may be used, it is preferred that the paper be selected from the group consisting of bleached Kraft paper and bleached sulfite paper. If no other paper layer is to be utilized, the white paper will be selected to have adequate rigidity to provide structural strength to the finished tube for its use as a tampon applicator. The paper may contain additional optical brighteners, e.g., stilbene derivatives, and the like to enhance the aesthetic appearance of the laminate. The brighteners, and overall whiteness of the paper, work in conjunction with the gloss of the cellophane layer to greatly improve the aesthetic appearance of the resulting applicator. Preferred thicknesses for the outer paper layer are in the range of 0.0015 (3,81.10⁻⁵m) to 0.005 (12,7.10⁻⁵m) inches, more preferably 0.003 inches (7,6.10⁻⁵m).

Other polymers may be used in place of cellophane, although with loss of some of the unique advantages of cellophane for this application. A variety of thermoplastic polymers may be used. The polymers should be capable of being formed into a relatively thin sheet, and be themselves water-resistant or have a coating or additive which imparts water-resistance. Preferred polymers include but are not limited to cellophane, polyethylene, polyester, polypropylene, polycaprolactone, and ethylene vinyl acetate.

The tampon holder may have no outer cellophane or polymer layer, provided it has adequate rigidity to withstand the knurling and/or circumferential-cut operations. Suitable laminates which do not have a cellophane or polymer layer should be at least about 0.010 (25,4.10⁻⁵m) inch thick, preferably 0.010 (25,4.10⁻⁵m) to 0.020 (50,8.10⁻⁵m) inch thick.

Any conventional process can be used to form the tube, e.g., spiral or convolute winding of the individual layers, each layer on top of the previous layer about a common central axis. Spiral winding is generally preferred. It is also preferred that the seams formed in each layer during spiral winding be offset from the seams in other layers. These methods are well known to those skilled in the art.

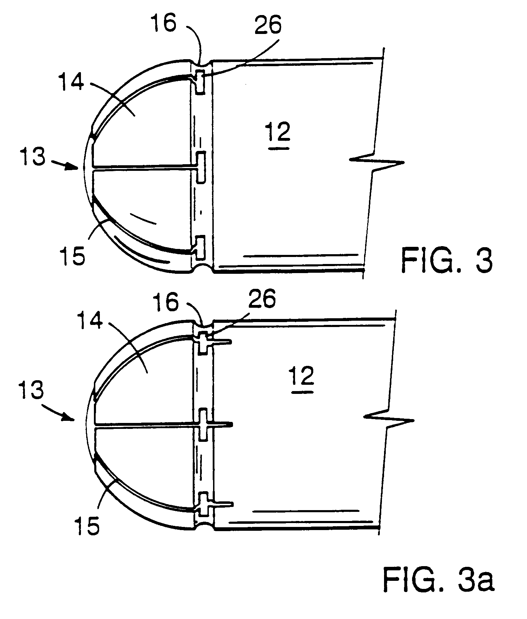

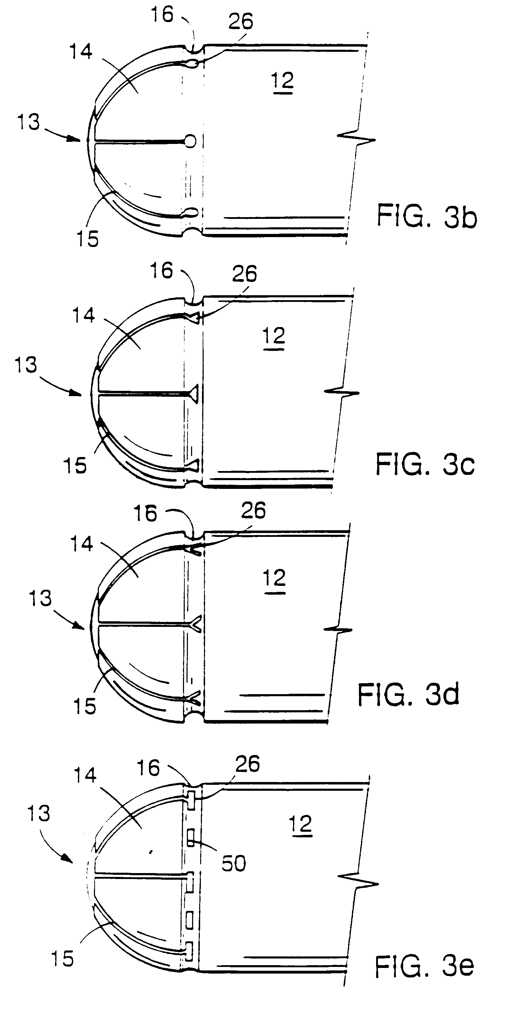

While preferred embodiments have been described above, other variations and modifications are within the scope of the following claims. As examples of the many possible variations, either the circumferential cuts or the score lines could be used by themselves, with or without groove 16 (or some other weakening formation). The slits separating the petal segments can extend beyond the hinge (e.g., as shown in Fig. 3a). The circumferential cuts and score lines need not be precisely aligned with one another, nor with groove 16 (or other weakening formation), although typically expulsion force is minimized if the features are aligned. The circumferential cuts need not extend exactly circumferentially, but could be cut at an angle to the circumferential direction (as shown in Fig. 3d); and other shapes such as circular and triangular cut outs (as shown in Figs. 3b and 3c) could be used, as long as they have some circumferential extent. The cuts need not actually remove material, as shown, but could simply be slits in the paper. The cuts need not be centered on the slits (as shown in Figs. 3 and 3a), but could be offset (as shown in Fig. 3e), and the alignment with slits could vary randomly from slit to slit (Fig. 3e). Additional cuts could be positioned intermediate the slits (as at 50 in Fig. 3e). Although a laminated paper construction has been disclosed, the invention could be applied to nonlaminated paper constructions, and to plastic applicators.

## Claims

1. A tampon applicator (10) for insertion of a tampon into a body cavity, comprising
a tampon holder tube (12) having a longitudinal axis and an expulsion and dimensioned for insertion into the body cavity, the tampon holder tube including interior and exterior surfaces,
a plurality of segments (14) integral with and extending from the expulsion end, each segment having a width and thickness, with slits (15) separating the segments, and
a circumferentially-extending weakening formation on the exterior of the holder tube, the formation defining a circumferential hinge (16) at which the segments bend open during expulsion, and
a plunger (20), telescopically and slidably mounted in the holder and adapted to expel the tampon from the holder when pushed manually into the holder
characterised in that
the tampon holder includes a zone of indentation (28) extending along said longitudinal axis on the inside surface of the tampon holder tube (12), and
the zone axially overlaps the weakening formation (16) on the exterior surface of the holder tube (12), thereby weakening the holder tube at the circumferential hinge and reducing the force required to expel the tampon.

## Patentansprüche

1. Tampon-Appliziervorrichtung (10) zum Einführen eines Tampons in einen Körperhohlraum, mit
einem Tamponhalterohr (12), das eine Längsachse und ein Austreibungende aufweist und zur Einführung in den Körperhohlraum bemessen ist, wobei das Tamponhalterohr umfaßt
einer inneren und einer äußeren Oberfläche,
einer Mehrzahl von Segmenten (14), die einstückig mit dem Austreibungsende und von diesem vorstehend ausgebildet sind, wobei jedes Segment eine Breite und eine Dicke aufweist, mit die Segmente trennenden Schlitzen (15), und
einer sich umfänglich erstreckenden Schwächungsformation auf der Außenseite des Halterohres, wobei die Formation ein Umfangsgelenk (16) definiert, an welchem sich die Segmente während der Austreibung aufbiegen, und
einem Kolben (20), der teleskopisch und gleitfähig in dem Halter angebracht ist und so ausgebildet ist, daß er den Tampon aus dem Halter austreibt, wenn er manuell in den Halter geschoben wird,
dadurch gekennzeichnet, daß
der Tamponhalter eine Vertiefungszone (28) umfaßt, die sich entlang seiner Längsachse auf der innenliegenden Oberfläche des Tamponhalterohres (12) erstreckt, und
die Zone die Schwächungsformation (16) auf der äußeren Oberfläche des Halterohres (12) axial überlappt, um dadurch das Halterohr an dem Umfangsgelenk zu schwächen und die zum Austreiben des Tampons erforderliche Kraft zu reduzieren.

## Revendications

1. Applicateur de tampon (10) pour l'insertion d'un tampon dans une cavité corporelle, comprenant :
un tube support de tampon (12) ayant un axe longitudinal et une extrémité d'expulsion qui est dismensionnée pour être insérée dans la cavité corporelle, le tube support de tampon comprenant :
des surfaces intérieures et extérieures,
une pluralité de segments (14) solidaires de l'extrémité d'expulsion et s'étendant à partir de cette dernière, chaque segment ayant une certaine largeur et une certaine épaisseur, avec des fentes (15) séparant les segments, et
une formation d'affaiblissement s'étendant dans la direction circonférentielle sur l'extérieur du tube support, la formation définissant une articulation circonférentielle (16) au niveau de laquelle les segments s'ouvrent par pliage pendant l'expulsion, et
un plongeur (20) monté de manière télescopique et coulissante dans le support, et conçu pour expulser le tampon du support lorsqu'il est poussé manuellement dans le support
caractérisé en ce que
le support de tampon comprend une zone d'entailles (28) s'étendant le long dudit axe longitudinal sur la surface intérieure du tube support de tampon (12), et
la zone chevauche la formation d'affaiblissement (16) dans la direction axiale, sur la surface extérieure du tube support (12), ce qui affaiblit le tube support au niveau de l'articulation circonférentielle et réduit la force nécessaire pour expulser le tampon.
